# EUROPEAN PATENT SPECIFICATION

(11) **EP 3 331 442 B1**
(45) Date of publication and mention of the grant of the patent: **09.12.2026**
(21) Application number: 16748236.3
(22) Date of filing: 27.07.2016
(51) Int. Cl.: A61B 5/15, A61B 5/153

(54) **APPARATUS FOR RAPID COLLECTION OF BLOOD FROM LIVESTOCK**
GERÄT ZUM SCHNELLEN ENTNEHMEN VON BLUT VON VIEH
APPAREIL POUR LE PRÉLÈVEMENT RAPIDE DE SANG DES BÉTAIL

(30) Priority: 29.07.2015 US 201562198402 P
(43) Date of publication of application: 13.06.2018
(73) Proprietor: Advanced Animal Diagnostics, Inc., Morrisville NC 27560 (US)
(72) Inventor: SINK, John Richard, Raleigh, North Carolina 27614 (US); POLLARD, Jasper N., Durham, North Carolina 27712 (US); PENA, Gustavo, Canyon, Texas 79015 (US); ASION, Deborah, Durham, North Carolina 27703 (US); HOCKETT, Mitchell, Raleigh, North Carolina 27617 (US); DRACH, Joy Parr, Pontiac, Illinois 61764 (US); PAYNE, Martha, Hillsborough, North Carolina 27278 (US); CALDERWOOD, David A., Chapel Hill, North Carolina 27517 (US)
(74) Representative: Barker Brettell LLP
(86) International application number: PCT/US2016/044210
(87) International publication number: WO 2017/019743

(56) References cited:
- EP-A1- 1 740 096
- EP-A2- 0 047 176
- WO-A2-2005/011773
- DE-A1- 4 132 480
- US-A- 3 747 812
- US-A- 4 133 304
- US-A- 4 595 021
- US-A- 5 919 356
- US-B2- 8 092 394

## Description

### Field of the Invention

The present invention concerns apparatus for rapidly collecting blood from a subject, particularly veterinary subjects such as cattle and other livestock, and rapidly dispensing that blood into a receptacle for further analysis.

### Background of the Invention

The world cattle population is estimated to be about 1.3 billion head, with about 30% in Asia, 20% in South America, 15% Africa, 14% in North and South America and 10% in Europe. Cattle inventory in the United States as of January 2015 was estimated at 89.9 million head, living on upwards of 600,000 farms and ranches. Cumulatively, the beef industry has an economic impact of $44 billion.

Approximately 20% of any one feedlot's cattle will become sick with infection. It is difficult to identify which cattle are sick and preventative diagnostic measures are lacking. As a result, cattle considered to be at high risk will typically be treated with antibiotics. To illustrate how widespread this practice has become, a recent report found agriculture accounts for 80% of antibiotic sales. With public concern mounting against the overuse of antibiotics in livestock, ranchers and farmers are forced to choose between medicating healthy cattle or risk pathogenic exposure to their entire herd.

The cattle chute at the feedyard provides convenient access to individual animals, but the amount of time each animal is available for withdrawal of a blood sample while in the chute is short. Accordingly, development of methods and apparatus for rapid withdrawal of blood from cattle as they pass through the chute, which blood can then be used for individual detection of infection, allowing for treatment on an animal-by-animal (rather than whole-herd) basis, is urgently needed.

US 5 919 356 A discloses a portable device for sampling fluid. The device has a puncture device attached to a housing which has an interior and exterior wall. The housing is in fluid communication with the syringe and contains a filtration device comprised of at least one U-shaped hollow fiber membrane. The device also includes a collection chamber. Also in the device is a sensing device including a further hollow fiber membrane the first and second ends each connected to a first and second plug. Each of the plugs contacts the interior wall of the housing. The further hollow fiber membrane contains a sensing agent. The device is arranged so that a sample is collected through the puncture device and is passed through the filtration device where it is passed through the one or more U-shaped hollow fiber membrane to the collection chamber. From the collection chamber the sample is passed through the hollow fiber membrane which contains the sensing agent device before being passed to a syringe.

### Summary of the Invention

Initially, we attempted to develop apparatus and methods for withdrawal of blood from the animal's ear. These techniques were found to be too slow (albeit by a matter of tens of seconds), and insufficiently reliable. In addition, blood flow to the ear is reduced by chilling, making these procedures less reliable in cold weather.

We also proposed methods and apparatus for withdrawal of blood from the animal's snout. Initial testing seemed successful, but the possible risk of infection when the animal is subsequently feeding (with its snout often contacting food shared with other animals) made such techniques unacceptable to some feedlots.

After further efforts, we developed, and herein describe, an apparatus useful for the rapid collection of blood from a subject (e.g., the vein of a bovine subject) and dispensing the blood therefrom into a collection device. The apparatus according to the invention is defined in claim 1. The apparatus comprises:
*(a)* a capillary body having a capillary chamber formed therein, with the chamber having an inlet and outlet;
*(b)* a hollow barrel connected to the chamber outlet;
*(c)* an ejector operatively associated with the barrel (e.g. a plunger positioned in the barrel, a squeezable bulb connected to the barrel, or other ejector device operatively associated with the barrel); and
*(d)* a hollow needle connected to the capillary body, with the hollow needle in fluid communication with the capillary chamber through the capillary inlet, wherein said chamber is configured to draw blood therein through said inlet by capillary action or by a combination of capillary action and pressure;
*(e)* a hub member, wherein both said hollow needle and said capillary body are connected to said hub member;

and wherein said capillary body and said hollow needle are removably connected to one another through linear frictional engagement through the hub member, with said capillary body being removably connected to said hub member,
such that, in use, blood can be rapidly drawn from the vein, artery or capillary bed of the subject into said capillary chamber through said needle, then the linear frictional disconnection of the capillary body from the hub member facilitates rapid removal of the hub member and the needle together from the capillary body, and then blood is dispensed from the capillary body into a collection device by activating said ejector,
wherein the apparatus further comprises:
   *(f)* a vent opening formed in either said barrel or said ejector, with said capillary chamber in fluid communication with said vent opening.

Therefore blood can be rapidly drawn into the capillary chamber through the needle by the combined action of vein pressure (or other pressure, if present, such as arterial pressure) and capillary action. The needle is removed and blood dispensed from the capillary body into a collection device by actuating the ejector (*e.g*., depressing a plunger).

The invention also provides a method of collecting a blood sample from a subject. The method according to the invention is defined in claim 12. The method comprises the steps of:
(a) inserting the hollow needle of an apparatus according to the invention into said subject for a time sufficient for said capillary chamber to at least partially fill with said blood sample;
(b) withdrawing said needle from the subject;
(c) separating the hub member of the apparatus and said needle together from said capillary chamber;
   and then
(d) dispensing said blood sample from said capillary chamber into a collection device by actuating the ejector of the apparatus.

The present invention is explained in greater detail in the specification below, which sets for non-limiting examples and embodiments. While the invention is explained primarily with reference to its primary purpose of collecting jugular vein blood from bovines such as cattle, it will be appreciated that the invention can be used on other human or animal subjects (particularly veterinary and livestock subjects such as rabbits, goats, sheep, horses, pigs, etc.) and can be used to collect blood from other locations (*e.g*., other veins such as the cephalic vein, saphenous vein, ear vein, milk vein (*i.e.,* the subcutaneous abdominal vein), tail vein (*i.e.,* the coccygeal vein), median cubital vein, etc., as well as blood from capillary beds and arteries).

### Brief Description of the Drawings

**Figure 1** is perspective view of an example capillary pipette and plunger subassembly for use in carrying out the present invention.
**Figure 2** is an exploded perspective view of a capillary pipette, connector, and needle assembly of an example embodiment of the present invention.
**Figure 3** is a perspective view of the combination of Figure 2, with the separate components assembled together, and ready for use in collecting blood from the jugular vein of a cow.
**Figure 4** is a perspective view of the combination of Figure 3, with the needle and connector hub removed, so that blood can be rapidly dispensed from the pipette and plunger subassembly into a blood sample receptacle for further analysis.
**Figure 5** **is** a perspective view of one embodiment of a one way valve, or check valve, positioned in a device of Figures 2-4.

### Detailed Description of Illustrative Embodiments

The present invention now will be described more fully hereinafter with reference to the accompanying figures, in which embodiments of the invention are shown. This invention may, however, be embodied in many alternate forms and should not be construed as limited to the embodiments set forth herein. Like numbers refer to like elements throughout the description of the figures.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the present application and relevant art and should not be interpreted in an idealized or overly formal sense unless expressly so defined herein. The terminology used in the description of the invention herein is for the purpose of describing particular embodiments only and is not intended to be limiting of the invention.

Also as used herein, "and/or" refers to and encompasses any and all possible combinations of one or more of the associated listed items, as well as the lack of combinations when interpreted in the alternative ("or").

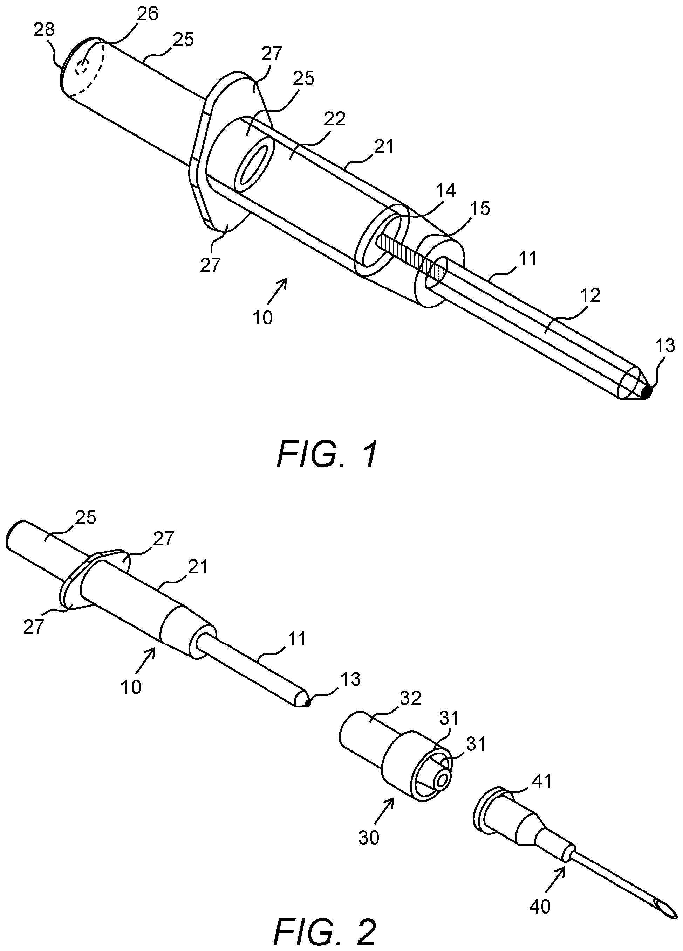

**Figures 1-4** show a non-limiting example of present invention. As noted above, the apparatus comprises, in overview, a capillary collection and dispensing subassembly **10,** a connecting hub **30,** and a hollow needle **40.**

The capillary collection and dispensing subassembly generally includes a capillary body **11** having a capillary chamber **12** formed therein, the chamber having an inlet **13** and outlet **14,** with the chamber configured to draw blood therein through the inlet by capillary action. A hollow barrel **21** is connected to the capillary chamber outlet, a hollow barrel connected to the chamber outlet. The barrel has a barrel chamber **22,** and a plunger **25** is positioned in the barrel.

The hollow needle **40** is removably connected to the capillary body, with the hollow needle in fluid communication with the capillary chamber through the inlet **13,** so that blood can be rapidly drawn into the capillary chamber through the needle **40** by the combined action of vein (or artery, or capillary bed) pressure (when present) and capillary action, the needle removed, and then blood dispensed into a collection device (not shown) through inlet **13** by depressing the plunger **25.**

There is a vent opening **26** formed in either the barrel or the plunger (or bulb, when a bulb is used in place of a plunger), with the capillary chamber in fluid communication with the vent opening, to facilitate capillary flow into the capillary chamber. In a particularly preferred embodiment, the vent opening is formed in the plunger end portion, or similarly configured on a squeezable bulb, so that it can be covered and blocked by the user's thumb or other digit when the blood is ejected when the plunger is depressed or ejector is actuated.

Preferably, the barrel has at least one laterally projecting grasping projection **27** connected thereto, to facilitate handling by a human user attempting to draw a blood sample from a (sometimes large) animal.

Preferably, the plunger has a blunt end portion **28** configured for depression of the plunger into the barrel by the user, and wherein the vent opening is formed in the blunt end portion *(e.g.,* to facilitate one-handed operation during dispensing, as discussed below).

The capillary chamber, the barrel, the plunger or ejector, and the needle may all be axially aligned with one another to facilitate handling, the other geometries may also be employed (*e.g*., angled geometries).

The apparatus further comprises a hub member **30,** with both the hollow needle and the capillary body connected to the hub member, and withd the capillary body being removably connected to the hub member. Numerous alternate configurations of the hub member can be implemented, as discussed further below. In the illustrated embodiment, both the capillary body and the hollow needle are removably connected to the hub member, the one with linear frictional engagement, and the other by twisting frictional engagement aided by the Luer lock fitting as discussed below.

Preferably at least a portion of the capillary body is transparent, and the apparatus includes a porous indicator element **15** *(e.g.,* a cotton, synthetic fiber, or glass fiber filter) positioned in the capillary chamber outlet opening adjacent a transparent portion of the capillary body. During use, when the indicator element shows at least partial saturation with blood, the operator knows that the capillary chamber **12** has been sufficiently filled and may withdraw the device from the animal.

In some embodiments, the capillary chamber has a volume of from 10 microliters to 50, 100 or 200 microliters. In some specific embodiments, the capillary chamber has a volume of 20 microliters. In other embodiments, the capillary chamber has a volume of 50 or 100 microliters to 200, 500, or 1000 microliters, or more.

In some embodiments, particularly those suitable for larger animals, the hollow needle **40** has a length of from 10 or 15 millimeters to 30 or 40 millimeters (preferably 25 millimeters), and/or the hollow needle is between 14 or 16 gauge and 20 or 22 gauge (preferably 18 gauge). For smaller animals/subjects, the needle may have a length of from 5 or 10 millimeters to 10 or 20 millimeters, and/or may be between 8 or 10 gauge and 14 or 18 gauge. In some embodiments, the hollow needle **40** has at least one lateral vent *(e.g.,* holes, slots, etc., not shown) formed therein and configured to facilitate rapid blood collection.

In the claimed invention, the capillary body and the hollow needle are removably connected to one another through linear frictional engagement through the hub member **30.**

Such a hub member **30** member may comprise at least one laterally projecting grasping member **31** connected thereto, preferably positioned in opposing relation to the grasping member **27** on the barrel, to facilitate rapid removal of the needle from the capillary body.

The capillary chamber may contain a reagent composition in liquid (generally aqueous, including gels) or dry form, such as anticoagulants, chelating agents, stains, dyes, etc.

In some embodiments, an anticoagulant or chelating agent *(e.g.,* a salt of ethylenediaminetetraacetic acid (EDTA) in dry powder form) is contained in the chamber **13** in a predetermined amount: That is, an amount appropriate for subsequent diagnostic testing, based on the volume of the capillary chamber, with larger amounts being included when the capillary chamber is large, and smaller amounts included when the capillary chamber is small. Specific amounts are known or readily determined by those skilled in the art based on the particular test being performed and testing apparatus being used. In those embodiments where the needle is not removable, an anticoagulant may be included inside the needle to treat blood which does not progress up into the capillary chamber where the bulk of the anticoagulant resides.

Any suitable anticoagulant or chelating agent may be used, preferably in dry or dry particulate form. In some embodiments of the invention, the one or more anticoagulants may be abciximab (ReoPro), acenocoumarol, antithrombin III, argatroban, aspirin, bivalirudin (Angiomax), clopidogrel, dabigatran, dabigatran etexilate (Pradaxa/Pradax), desirudin (Revasc/Iprivask), dipyridamole, eptifibatide (Integrilin), fondaparinux, heparin, hirudin, idraparinux, lepirudin (Refludan), low molecular weight heparin, melagatran, phenindione, phenprocoumon, ticlopidine, tirofiban (Aggrastat), warfarin, ximelagatran, ximelagatran (Exanta/Exarta), or any combination thereof. See e.g. US 9,085,615.

In particular embodiments of the invention, the one or more anti-coagulants may be chelators of di-valent cations selected from Ethylenediaminetetraacetic acid (EDTA), Ethylene glycol tetraacetic acid (EGTA), FURA-2, or a combination thereof, particularly dry particulate salts thereof.

In the example embodiment of **Figure 1-4****,** a suitable barrel and capillary body subassembly, including plunger and indicator plug, is a MINIVETTE^{®} POCT 20 microliter capillary blood collection device, containing a defined or predetermined amount of EDTA tripotassium salt, Reference number 17.2113.120, available from Sarstedt Aktiengesellschaft & Co. A suitable hollow needle is a BD PRECISIONGLIDE^{™} 18G x 1 (1.2mm x 25mm) single use needle, Reference number 305195, available from Becton, Dickinson and Company. A suitable hub member is formed from an EXELINT^{®} 1 milliliter Luer lock tip disposable syringe, reference number 26050, available from EXEL International, with the plunger discarded and the upper portion of the barrel cut off, leaving the lower portion of the barrel **32** to guide insertion of the capillary body **12** during assembly. Note that the foregoing includes a Luer lock fitting **31,** which both cooperates in receiving the corresponding Luer lock fitting **41** on the needle, and which conveniently forms the projecting/grasping element (in this case an annular grasping element) described above.

Those skilled in the art will appreciate numerous variations to the foregoing. For example, the hub member **31** can be a custom-molded part, rather than cut from a syringe body. One or more projecting members on the hub can be enlarged and shaped to be more readily grasped by a user, rather than fortuitously formed from the angled exterior intersection of the syringe barrel and the Luer lock tip thereon. The hub can be permanently connected to the hypodermic needle.

In addition, while the example embodiment of **Figures 1-4** has two removable connections (the first a Luer lock connection of the needle to the hub via corresponding Luer lock fittings **31,41;** the second the linear frictional connection of the **30** hub to the capillary blood collection device **10**), when a single removable connection is employed it is preferably a linear frictional connection to facilitate rapid removal of the needle from the capillary collection subassembly (although, in a still further embodiment, projecting "wings" can be formed on the hub and/or capillary collection subassembly, to facilitate separation of the two by rapid twisting by the user).

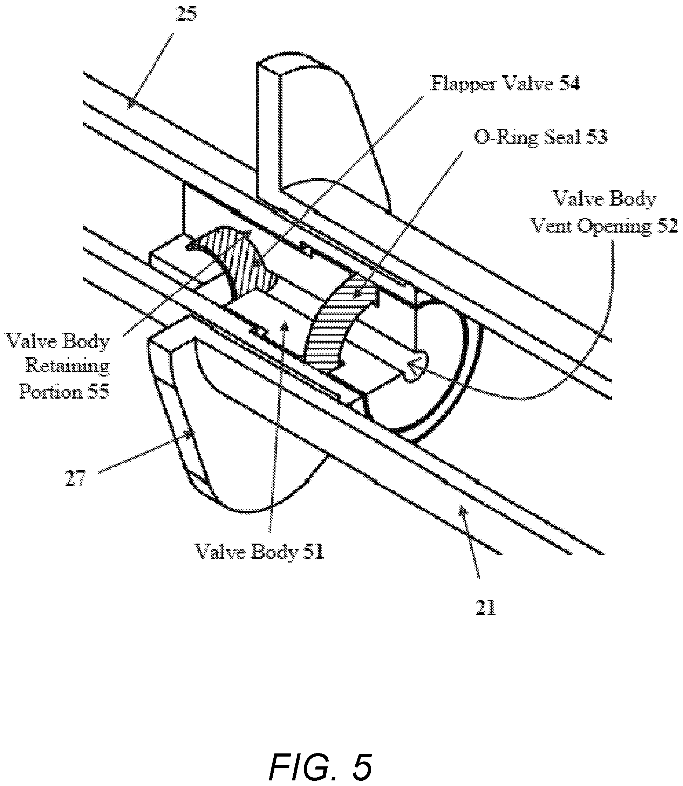

**Figure 5** illustrates one embodiment of a device of the invention incorporating a one-way valve, or check valve. The purpose of the check valve is to inhibit the backflow of collected blood from the device back into the animal, particularly when the device contains liquid or dry reagents in the capillary chamber for mixing with the collected blood. In the illustrated embodiment, the check valve comprises a valve body **51** inserted into the hollow plunger **25.** The valve body may be formed of any suitable material, such as polymer injection molded or fabricated by additive manufacturing. The valve body includes a vent opening **52,** and may include an O-ring seal **53** (or other suitable sealing features) to seal the valve body to the interior wall of the hollow plunger. The valve itself may be a flapper valve **54,** as illustrated, formed from any suitable material, such as glove latex or a sheet of silicone rubber, which in the illustrated embodiment is affixed into the valve body **51** in a valve body retaining portion **55,** which valve body retaining portion is configured to geometrically retain the flapper valve in the valve body without the need for adhesive or other fastener. Numerous alternatives for the position of the check valve are available, including above the vent opening 28, within the plunger 25, as shown, within barrel chamber 22 (so long as sufficient clearance for the plunger is provided), between barrel chamber 22 and capillary chamber 12, between capillary chamber 12 and hollow needle 40, etc. (though in some of these embodiments, the check valve would preferably be configured to overcome by relatively high plunger pressure to expel blood).

In preferred embodiments, the check valve is positioned on the "gas side" or "vent side" above, or upstream from, the barrel chamber (*i.e.,* above the vent opening or within the plunger, or between the barrel and a squeezable bulb).

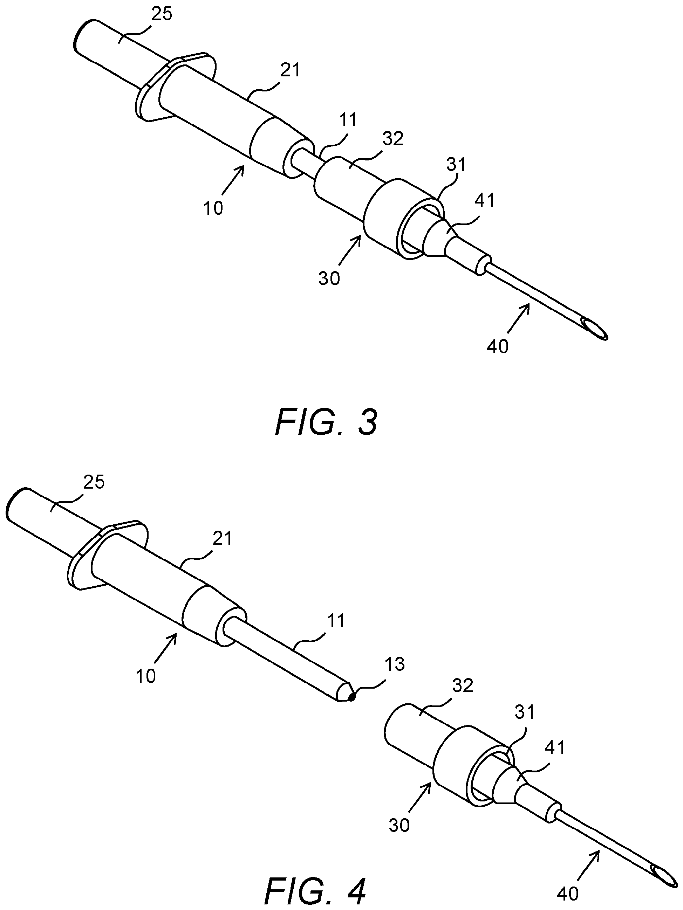

***Methods of use.*** Devices as described above may be used to collect blood from a subject by:
*(a)* inserting the hollow needle of an apparatus as described above (*see, e.g.,* Figure 3) into the subject for a time sufficient for said capillary chamber to at least partially fill with said blood sample;
(b) withdrawing the needle from the subject;
(c) separating the needle from the capillary chamber (*see, e.g.,* Figure 4); and then
*(d)* dispensing the blood sample from the capillary chamber into a collection device *(e.g.,* from the capillary chamber, or through the needle when the needle is not separated from the chamber) by depressing said plunger, squeezing the bulb, or otherwise actuating the ejector.

In some embodiments, the subject is a bovine (including bulls, steers, cows, and calves), although the method may be applied to other subjects as noted above.

In some embodiments, the needle is inserted into a vein *(e.g.,* the jugular vein) of the subject, although other locations may sometimes be used as well, particularly tissues rich in capillary beds.

In some embodiments, the procedure is carried out rapidly, for example, with steps (a) through (d) are being carried out in a time of one minute or less, or even thirty or twenty seconds or less *(e.g.,* an average time of one minute, or thirty or twenty seconds, or less, when blood is collected sequentially from two or more subjects). When time is not a major concern, the procedure may be carried out more slowly.

Dispensing of the blood sample may be into any suitable collection device or receptacle for further analysis. Numerous alternatives are known in the art, with particular examples including, but not limited to, those described in US Patent No. 9,023,641, and in US Patent Application Publication Nos. 2014/0315242, 2014/0315283, and 2014/0186859.

While the invention has been described above with a pull-type plunger, note that the plunger can be used as a push-pull type plunger, which would facilitate blood collection from certain sources such as a tail vein. For such a use, additional elements *(e.g.,* rings, tabs, etc.) can be added to the plunger to facilitate pulling by the user.

In addition, while the invention has been described above with a rigid capillary portion, the capillary portion may alternatively be formed of a flexible material, for example, to facilitate ejection of the blood sample by squeezing the capillary portion.

The foregoing is illustrative of the present invention, and is not to be construed as limiting thereof. The invention is defined by the following claims.

## Claims

1. An apparatus useful for the rapid collection of blood from the vein, artery or capillary bed of a subject and dispensing the blood therefrom into a collection device,
the apparatus comprising:
*(a)* a capillary body (11) having a capillary chamber (12) formed therein, said chamber having an inlet (13) and outlet (14);
*(b)* a hollow barrel (21) connected to said chamber outlet;
*(c)* an ejector operatively associated with said barrel;
*(d)* a hollow needle (40) connected to said capillary body, with said hollow needle in fluid communication with said capillary chamber through said inlet;
wherein said chamber is configured to draw blood therein through said inlet by capillary action or by a combination of capillary action and pressure;
*(e)* a hub member (30), wherein both said hollow needle and said capillary body are connected to said hub member;
and wherein said capillary body and said hollow needle are removably connected to one another through linear frictional engagement through the hub member, with said capillary body being removably connected to said hub member,
such that, in use, blood can be rapidly drawn from the vein, artery or capillary bed of the subject into said capillary chamber through said needle, then the linear frictional disconnection of the capillary body from the hub member facilitates rapid removal of the hub member and the needle together from the capillary body, and then blood is dispensed from the capillary body into a collection device by activating said ejector,
**characterized in that** the apparatus further comprises:
*(f)* a vent opening (26) formed in either said barrel or said ejector, with said capillary chamber being in fluid communication with said vent opening.

2. The apparatus of claim 1, wherein the hollow needle is removably connected to the hub member.

3. The apparatus of claim 1, wherein said ejector comprises a plunger (25) having a blunt end portion (28) configured for depression of said plunger into said barrel, and wherein said vent opening is formed in said blunt end portion.

4. The apparatus of any one of claims 1 to 3, wherein said barrel has at least one laterally projecting grasping projection (27) connected thereto.

5. The apparatus of any one of claims 1 to 4, wherein:
a) said capillary chamber, said barrel, said ejector, and said needle are all axially aligned with one another; and/or
b) said capillary chamber has a volume of from 10 to 50 microliters.

6. The apparatus of any one of claims 1 to 5, wherein said hub member comprises at least one laterally projecting grasping member connected thereto.

7. The apparatus of any one of claims 1 to 6, further comprising:
a) a chelating agent contained in said capillary chamber in a predetermined amount; and/or
b) a liquid or gel reagent composition in said capillary chamber in a predetermined amount.

8. The apparatus of claim 7, option a), wherein said chelating agent is in dry powder form.

9. The apparatus of any one of claims 1 to 8, further comprising a one-way valve operatively associated with said capillary chamber and/or vent opening said one-way valve configured to prevent backflow of collected blood from the capillary chamber back into the animal.

10. The apparatus of any one of claims 1 to 9, wherein at least a portion of said capillary body is transparent, said capillary body further comprising a porous indicator element (15) positioned in said capillary chamber outlet opening adjacent a transparent portion of said capillary body.

11. The apparatus of any one of claims 1 to 10, wherein said hollow needle:
a) has a length of from 10 to 40 millimeters; and/or
b) is between 14 or 16 gauge and 20 or 22 gauge.

12. A method of collecting a blood sample from a subject, comprising the steps:
*(a)* inserting the hollow needle of an apparatus as defined in any one of claims 1 to 11 into said subject for a time sufficient for the capillary chamber of the apparatus to at least partially fill with said blood sample;
*(b)* withdrawing said needle from the subject;
*(c)* separating the hub member of the apparatus and said needle together from said capillary chamber;
and then
*(d)* dispensing said blood sample from said capillary chamber into a collection device by actuating the ejector of the apparatus.

13. The method of claim 12, wherein said subject is a bovine.

14. The method of claim 12 or 13, wherein said needle is inserted into a vein of the subject.

15. The method of any one of claims 12 to 14, wherein said needle is inserted into tissues rich in capillary beds.

## Patentansprüche

1. Vorrichtung, die zur schnellen Sammlung von Blut aus der Vene, Arterie oder dem Kapillarbett eines Subjekts nützlich ist und das Blut daraus in eine Sammeleinrichtung abgibt, wobei die Vorrichtung Folgendes umfasst:
*(a)* einen Kapillarkörper (11), der eine darin gebildete Kapillarkammer (12) aufweist, wobei die Kammer einen Einlass (13) und einen Auslass (14) aufweist;
*(b)* einen hohlen Zylinder (21), der mit dem Kammerauslass verbunden ist;
*(c)* einen Auswerfer, der betriebswirksam dem Zylinder zugeordnet ist;
*(d)* eine Hohlnadel (40), die mit dem Kapillarkörper verbunden ist, wobei die Hohlnadel durch den Einlass in Fluidkommunikation mit der Kapillarkammer steht;
wobei die Kammer dazu konfiguriert ist, durch Kapillarwirkung oder durch eine Kombination aus Kapillarwirkung und Druck Blut durch den Einlass dort hineinzusaugen;
*(e)* ein Nabenelement (30), wobei sowohl die Hohlnadel als auch der Kapillarkörper mit dem Nabenelement verbunden sind;
und wobei der Kapillarkörper und die Hohlnadel durch linearen Reibschluss durch das Nabenelement entfernbar miteinander verbunden sind, wobei der Kapillarkörper entfernbar mit dem Nabenelement verbunden ist,
sodass bei Verwendung Blut schnell aus der Vene, Arterie oder dem Kapillarbett des Subjekts durch die Nadel in die Kapillarkammer gezogen werden kann, dann die Trennung des linearen Reibschlusses des Kapillarkörpers von dem Nabenelement eine schnelle Entfernung des Nabenelements und der Nadel gemeinsam aus dem Kapillarkörper erleichtert und dann Blut durch Aktivieren des Auswerfers aus dem Kapillarkörper in eine Sammeleinrichtung abgegeben wird,
**dadurch gekennzeichnet, dass** die Vorrichtung ferner Folgendes umfasst:
*(f)* eine Entlüftungsöffnung (26), die entweder in dem Zylinder oder dem Auswerfer gebildet ist, wobei die Kapillarkammer in Fluidverbindung mit der Entlüftungsöffnung steht.

2. Vorrichtung nach Anspruch 1, wobei die Hohlnadel entfernbar mit dem Nabenelement verbunden ist.

3. Vorrichtung nach Anspruch 1, wobei der Auswerfer einen Kolben (25) umfasst, der einen stumpfen Endabschnitt (28) aufweist, der zum Eindrücken des Kolbens in den Zylinder konfiguriert ist, und wobei die Entlüftungsöffnung in dem stumpfen Endabschnitt gebildet ist.

4. Vorrichtung nach einem der Ansprüche 1 bis 3, wobei der Zylinder mindestens einen seitlich vorstehenden Greifvorsprung (27) aufweist, der damit verbunden ist.

5. Vorrichtung nach einem der Ansprüche 1 bis 4, wobei:
a) die Kapillarkammer, der Zylinder, der Auswerfer und die Nadel alle axial aneinander ausgerichtet sind; und/oder
b) die Kapillarkammer ein Volumen von 10 bis 50 Mikroliter aufweist.

6. Vorrichtung nach einem der Ansprüche 1 bis 5, wobei das Nabenelement mindestens ein seitlich vorstehendes Greifelement umfasst, das damit verbunden ist.

7. Vorrichtung nach einem der Ansprüche 1 bis 6, ferner umfassend:
a) einen Chelatbildner, der in der Kapillarkammer in einer vorbestimmten Menge enthalten ist; und/oder
b) eine flüssige oder gelartige Reagenzzusammensetzung in der Kapillarkammer in einer vorbestimmten Menge.

8. Vorrichtung nach Anspruch 7, Option a), wobei der Chelatbildner in Trockenpulverform vorliegt.

9. Vorrichtung nach einem der Ansprüche 1 bis 8, ferner umfassend ein Einwegventil, das der Kapillarkammer betriebswirksam zugeordnet ist, und/oder eine Entlüftungsöffnung, wobei das Einwegventil dazu konfiguriert ist, einen Rückfluss von gesammeltem Blut aus der Kapillarkammer zurück in das Tier zu verhindern.

10. Vorrichtung nach einem der Ansprüche 1 bis 9, wobei mindestens ein Abschnitt des Kapillarkörpers transparent ist und der Kapillarkörper ferner ein poröses Anzeigeelement (15) umfasst, das in der Kapillarkammerauslassöffnung benachbart zu einem transparenten Abschnitt des Kapillarkörpers positioniert ist.

11. Vorrichtung nach einem der Ansprüche 1 bis 10, wobei die Hohlnadel:
a) eine Länge von 10 bis 40 Millimetern aufweist; und/oder
b) zwischen 14 oder 16 Gauge und 20 oder 22 Gauge hat.

12. Verfahren zum Sammeln einer Blutprobe von einem Subjekt, umfassend die folgenden Schritte:
*(a)* Einführen der Hohlnadel einer Vorrichtung nach einem der Ansprüche 1 bis 11 in das Subjekt für eine Zeit, die ausreichend ist, damit sich die Kapillarkammer der Vorrichtung mindestens teilweise mit der Blutprobe füllt;
*(b)* Zurückziehen der Nadel aus dem Subjekt;
*(c)* Trennen des Nabenelements der Vorrichtung und der Nadel gemeinsam von der Kapillarkammer;
und dann
*(d)* Abgeben der Blutprobe aus der Kapillarkammer in eine Sammeleinrichtung durch Betätigen des Auswerfers der Vorrichtung.

13. Verfahren nach Anspruch 12, wobei das Subjekt ein Rind ist.

14. Verfahren nach Anspruch 12 oder 13, wobei die Nadel in eine Vene des Subjekts eingeführt wird.

15. Verfahren nach einem der Ansprüche 12 bis 14, wobei die Nadel in Gewebe eingeführt wird, die reich an Kapillarbetten sind.

## Revendications

1. Appareil utile pour la collecte rapide de sang à partir de la veine, de l'artère ou du lit capillaire d'un sujet et la distribution du sang à partir de celui-ci dans un dispositif de collecte, l'appareil comprenant :
*(a)* un corps capillaire (11) ayant une chambre capillaire (12) formée dans celui-ci, ladite chambre ayant une entrée (13) et sortie (14) ;
*(b)* un cylindre creux (21) raccordé à ladite sortie de chambre ;
*(c)* un éjecteur associé de manière fonctionnelle audit cylindre ;
*(d)* une aiguille creuse (40) raccordée audit corps capillaire, avec ladite aiguille creuse en communication fluidique avec ladite chambre capillaire à travers ladite entrée ;
dans lequel ladite chambre est configurée pour l'aspiration de sang dans celle-ci à travers ladite entrée par action capillaire ou par une combinaison d'action capillaire et de pression ;
*(e)* un élément de moyeu (30), dans lequel à la fois ladite aiguille creuse et ledit corps capillaire sont raccordés audit élément de moyeu ;
et dans lequel ledit corps capillaire et ladite aiguille creuse sont raccordés de manière amovible l'un à l'autre à travers une mise en prise par friction linéaire à travers l'élément de moyeu, avec ledit corps capillaire étant raccordé de manière amovible audit élément de moyeu,
de telle sorte que, lors de l'utilisation, du sang peut être rapidement aspiré à partir de la veine, de l'artère ou du lit capillaire du sujet dans ladite chambre capillaire à travers ladite aiguille, puis la séparation par friction linéaire du corps capillaire par rapport à l'élément de moyeu facilite le retrait rapide de l'élément de moyeu et de l'aiguille ensemble à partir du corps capillaire, et ensuite du sang est distribué à partir du corps capillaire dans un dispositif de collecte par l'activation dudit éjecteur,
**caractérisé en ce que** l'appareil comprend en outre :
*(f)* une ouverture d'évent (26) formée dans l'un ou l'autre dudit cylindre ou dudit éjecteur, avec ladite chambre capillaire étant en communication fluidique avec ladite ouverture d'évent.

2. Appareil selon la revendication 1, dans lequel l'aiguille creuse est raccordée de manière amovible à l'élément de moyeu.

3. Appareil selon la revendication 1, dans lequel ledit éjecteur comprend un piston plongeur (25) ayant une portion d'extrémité contondante (28) configurée pour l'enfoncement dudit piston plongeur dans ledit cylindre, et dans lequel ladite ouverture d'évent est formée dans ladite portion d'extrémité contondante.

4. Appareil selon l'une quelconque des revendications 1 à 3, dans lequel ledit cylindre a au moins une saillie de préhension faisant saillie latéralement (27) raccordée à celui-ci.

5. Appareil selon l'une quelconque des revendications 1 à 4, dans lequel :
a) ladite chambre capillaire, ledit cylindre, ledit éjecteur, et ladite aiguille sont tous alignés axialement les uns avec les autres ; et/ou
b) ladite chambre capillaire a un volume allant de 10 à 50 microlitres.

6. Appareil selon l'une quelconque des revendications 1 à 5, dans lequel ledit élément de moyeu comprend au moins un élément de préhension faisant saillie latéralement raccordé à celui-ci.

7. Appareil selon l'une quelconque des revendications 1 à 6, comprenant en outre :
a) un agent chélateur contenu dans ladite chambre capillaire en une quantité prédéterminée ; et/ou
b) une composition de réactif liquide ou en gel dans ladite chambre capillaire en une quantité prédéterminée.

8. Appareil selon la revendication 7, option a), dans lequel ledit agent chélateur est sous forme de poudre sèche.

9. Appareil selon l'une quelconque des revendications 1 à 8, comprenant en outre une soupape unidirectionnelle associée de manière fonctionnelle à ladite chambre capillaire et/ou ouverture d'évent ladite soupape unidirectionnelle configurée pour la prévention d'un reflux de sang collecté à partir de la chambre capillaire de retour dans l'animal.

10. Appareil selon l'une quelconque des revendications 1 à 9, dans lequel au moins une portion dudit corps capillaire est transparente, ledit corps capillaire comprenant en outre un élément indicateur poreux (15) positionné dans ladite ouverture de sortie de chambre capillaire de manière adjacente à une portion transparente dudit corps capillaire.

11. Appareil selon l'une quelconque des revendications 1 à 10, dans lequel ladite aiguille creuse :
a) a une longueur allant de 10 à 40 millimètres ; et/ou
b) est entre un calibre 14 ou 16 et un calibre 20 ou 22.

12. Procédé de collecte d'un échantillon de sang à partir d'un sujet, comprenant les étapes consistant à :
*(a)* insérer l'aiguille creuse d'un appareil tel que défini dans l'une quelconque des revendications 1 à 11 dans ledit sujet pendant un temps suffisant pour que la chambre capillaire de l'appareil se remplisse au moins partiellement avec ledit échantillon de sang ;
*(b)* retirer ladite aiguille du sujet ;
*(c)* séparer l'élément de moyeu de l'appareil et ladite aiguille ensemble de ladite chambre capillaire ;
et ensuite
*(d)* distribuer ledit échantillon de sang à partir de ladite chambre capillaire dans un dispositif de collecte par l'actionnement de l'éjecteur de l'appareil.

13. Procédé selon la revendication 12, dans lequel ledit sujet est un bovin.

14. Procédé selon la revendication 12 ou la revendication 13, dans lequel ladite aiguille est insérée dans une veine du sujet.

15. Procédé selon l'une quelconque des revendications 12 à 14, dans lequel ladite aiguille est insérée dans des tissus riches en lits capillaires.
